# EUROPEAN PATENT SPECIFICATION

(11) **EP 2 681 211 B1**
(45) Date of publication and mention of the grant of the patent: **07.10.2015**
(21) Application number: 12712784.3
(22) Date of filing: 28.02.2012
(51) Int. Cl.: C07D 451/10, A61K 31/46, A61P 11/00

(54) **CRYSTALLINE COMPOUND COMPRISING TIOTROPIUM BROMIDE**
KRISTALLINE VERBINDUNG ENTHALTEND TIOTROPIUMBROMID
COMPOSÉ CRYSTALLINE COMPRENANT DU BROMURE DE TIOTROPIUM

(30) Priority: 28.02.2011 TR 201101897
(43) Date of publication of application: 08.01.2014
(73) Proprietor: Bilgic, Mahmut, 34220 Esenler-Istanbul (TR)
(72) Inventor: BLATTER, Fritz, CH-4303 Kaiseraugst (CH); RÖDEL, Eva, CH-4303 Kaiseraugst (CH)
(86) International application number: PCT/TR2012/000041
(87) International publication number: WO 2012/118460

(56) References cited:
- WO-A2-2006/117300
- WO-A2-2007/075838

## Description

The present invention refers to a crystalline compound comprising Tiotropium bromide and propionic acid and to a process for manufacturing the same.

Tiotropium bromide is disclosed in the application numbered EP 0 418 716 A1 and has the following chemical structure:

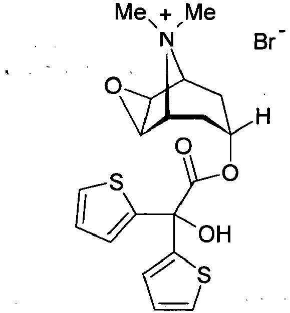

Tiotropium bromide is a highly effective anticholinegic with a long lasting effect. It can be used for treating respiratory complaints and particularly COPD (chronic obstructive pulmonary disease) and asthma.

For treating the abovementioned complaints, Tiotropium bromide is preferably administered by the inhalation route. In addition to the administration of broncholytically active compounds in the form of metered aerosols and inhalable solutions, use of inhalable powders containing active substance is of particular importance.

Tiotropium bromide is preferably administered by the inhalation route. Suitable inhalable powders packed into appropriate capsules (inhalettes) or blisters may be used. Alternatively, it may be administered by use of suitable inhalable aerosols. These also include powdered inhalable aerosols which contain, for example, HFA134a, HFA227 or mixtures thereof as propellent gas.

However, since Tiotropium bromide is very effective, only a small amount of the pharmaceutically active substance is needed per single dose to achieve the desired therapeutic effect.

Thus, the pharmaceutically active substance has to be diluted in a suitable excipient in order to prepare an inhalable powder.

The amount of inhalable composition must be accurately metered in every single dose.

Because of the large amount of excipient, the properties of the inhalable powder are significantly influenced by the choice of the excipient.

Moreover, the particle size of the pharmaceutically active substance and of the excipient is very important for the emptying characteristics of capsules or blisters when used in an inhaler.

The active substance particles that shall be administered by the inhalation route should optimally meet essential requirements such as appropriate aerodynamic particle size, appropriate particle shape, uniformity of particle size distribution, low aerodynamic dispersion forces, low density, high physical and chemical stability.

Additionally, other physical properties of the pharmaceutically active substance like absorption of water and ease of grinding of the pharmaceutically active substance are of great importance.

The correct manufacture of the abovementioned compositions which are suitable for use for the administration of a pharmaceutically active substance by inhalation is based on various parameters which are connected with the nature of the active substance itself. In pharmaceutical compositions which are used in the form of inhalable powders or inhalable aerosols like tiotropium bromide, the crystalline active substance is used in ground (micronised) form for preparing the formulation. Since the pharmaceutical quality of a pharmaceutical formulation requires that the pharmaceutically active substance should always have the same crystalline modification, the stability and properties of the crystalline active substance are subject to stringent requirements from this point of view as well.

Furthermore, micronised powders to be taken by the inhalation route which have moisture ratio as low as possible prevent the product from agglomerating and provide it to be transmitted into the target area more effectively. Therefore, one objective of the present invention is to develop crystalline forms which have lower moisture content and are more stable as compared to the known crystalline forms.

It is an objective of the present invention to provide Tiotropium bromide in salt form having good chemical and/or physical stability and/or good processability during both its preparation as a pharmaceutically active substance and in the preparation of pharmaceutical compositions containing Tiotropium bromide.

It is a further objective of the invention to provide crystalline salts of Tiotropium bromide that are accessible in a reproducible manner and constant quality in inexpensive and well controlled production processes.

The fact that tiotropium bromide has therapeutic efficacy as a pharmaceutically active substance even at very low doses indicates the need in the technical field to find other effective substances for administration in inhalable composition form. Thus, a further technical problem that the present invention relates to is to prepare practical dosage forms of Tiotropium bromide in inhalable composition form.

The technical problem underlying the present invention is solved by a crystalline compound comprising a mixture of a compound of formula I (Tiotropium bromide) 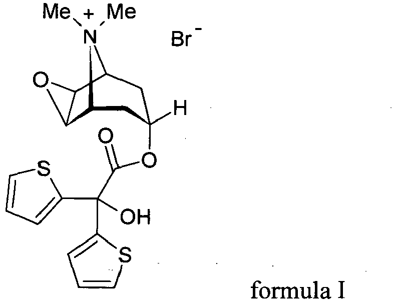 and propionic acid or a hydrate thereof, wherein the molar ratio of the compound of formula I and the propionic acid is in the range of from 1:0.3 to 1:0.7.

In the case that the crystalline compound comprises water, it is referred to be a hydrate.

The term "mixture of a compound of formula I (Tiotropium bromide) and propionic acid" signifies that these two substances are in the same crystalline matrix or different crystalline/amorphous matrices.

The crystalline compound of the present invention can be admixed with suitable excipients in order to obtain an inhalable composition. The crystalline compound can be ground in order to adjust the particle size precisely. Moreover, an inhalable composition comprising the crystalline compound of the present invention can be stored without losing effectiveness.

In addition, crystalline compounds according to the present invention are stable under accelerated conditions. It is possible to store the crystalline compound at a temperature of 40 °C without any decomposition or conversion. Consequently, the crystalline compound of the present invention can be used as medicament in countries generally having high temperatures. At elevated temperature the medicament will not lose effectiveness.

In a preferred embodiment of the present invention, the molar ratio of the compound of formula I in the crystalline compound and the propionic acid is in the range of 1: 0.45 to 1:0.55. Most preferred molar ratio of the compound of formula I and the propionic acid is 1:0.5.

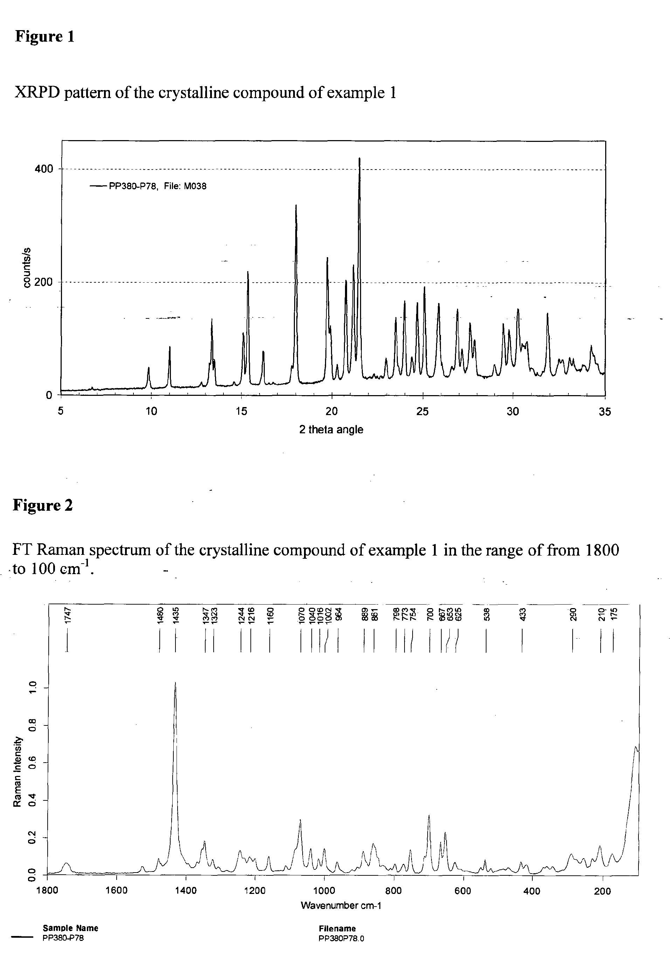

Preferably, the crystalline compound has a FT-Raman spectrum comprising peaks at wavenumbers (expressed in ± 2 cm⁻¹) of 3112, 3071, 2792, 1747, 1435, 1347, 1244, 1160, 1070, 1040, 964, 889, 861, 754, 700, 667, 653, 538, 433, 290 and 210 cm⁻¹. Preferably, the crystalline compound of the present invention can be further characterized by an FT Raman spectrum substantially in accordance with figure 2.

Preferably, the crystalline compound has an XRPD pattern with at least one characteristic peak (expressed in 2θ ± 0,1° 20 (CuKα radiation)) at 13.3°, 15.2°, 19.7°, 20.8°, 21.2°, 21.5°, 24.0°, 25.1°, 27.6°, 30.3°, 31.9° and 39.5°. Preferably, the crystalline compound of the present invention can be further characterized by an XRPD substantially in accordance with figure 1.

A further aspect of the present invention is a pharmaceutical composition comprising the crystalline compound of the present invention and optionally one or more pharmaceutically acceptable excipients.

The present invention is also directed to a process for obtaining the crystalline compound comprising the steps of:
a) providing a compound of formula I (Tiotropium bromide) 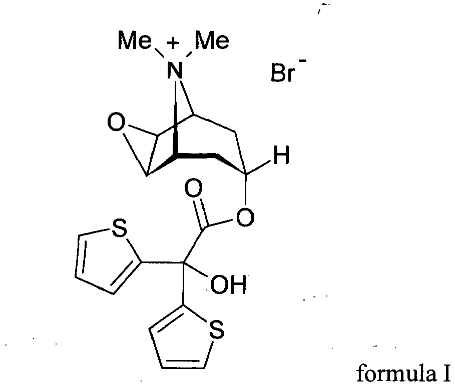
b) adding propionic acid to the composition of step a)
c) optionally concentrating the composition of step b)
d) crystallizing
e) optionally equilibrating the obtained suspension of step d) and
f) isolating the obtained precipitate.

Preferably, in step a), the compound of formula I is provided as solution in a suitable solvent or as a suspension/solution of the amorphous form of Tiotropium bromide in a suitable solvent.

In step b), neat propionic acid or propionic acid in a suitable solvent may be added to the composition of step a).

Suitable solvents are, for instance, polar and/or protic solvents such as ethanol, isopropanol, n-propanol, acetone, water, DMSO, ethyl acetate, acetonitrile, methyl ethyl ketone, tert butyl methyl ether or mixtures thereof. The solution of propionic acid in the solvent is preferably a saturated or nearly saturated solution.

In a further preferred embodiment of the invention, the molar ratio of the compound of formula I in step a) and propionic acid of step b) is in the range of from 1:0,5 to 1:10000, even more preferred of from 1:0,5 to 1:1000, preferably of from 1:0,5 to 1:500 and the most preferred is 1:100.

Said process may further comprise step g) of slurrying the isolated precipitate of step f) in a further suitable solvent or mixture of solvents and h) isolating the obtained precipitate.

Preferably, in step c), a part of the solvent is removed by for instance distillation or evaporation under reduced pressure or in a flow of a carrier gas like N₂.

Optionally, in step c), the entire solvent is removed by for instance distillation or evaporation under reduced pressure or in a flow of a carrier gas like N₂ and the residue is dissolved in a suitable solvent or mixture of solvents and water.

The solvent may be selected from a group consisting of C2-C4 alcohols, a C3-C6 ketone, an ether or an acetic ester C1-C4 alkylester, acetonitrile, a hydrocarbon or mixtures thereof.

In step d), e) and/or g); the obtained mixture may be stirred. Step d), e) and/or g) may be carried out at a temperature of from 10°C to 30°C. Suspension equilibration in step e) and/or slurrying in step g) are preferably carried out at a temperature from 10 °C to 30 °C and most preferred at ambient temperature. Step e) and/or g) are typically carried out for one day. Optionally, samples may be recovered from the suspension and analyzed by XRPD. Filtration is performed when the XRPD analysis indicates that the desired form is obtained in a reasonable purity.

Optionally, in step d), e) and/or g); seed crystals are added. Said seed crystals are preferably the desired form of the crystalline compound of the present invention which is to be obtained. However, it is an advantage of the present invention that adding of seed crystals is not generally necessary.

### Examples

### Instrumental Parameters and Measurement Procedures

### Powder X-ray diffraction (XRPD)

The measurements were carried out with a Bruker D8 Advance powder X-ray diffractometer using Cu Kα radiation in the Bragg-Brentano reflection geometry.

Generally, the 2θ values are accurate within an error of ±0.1-0.2°.

The relative peak intensities can vary considerably for different samples of the same crystalline form because of different preferred orientations of the crystals. The samples were prepared without any special treatment other than the application of slight pressure to get a flat surface. Silicon single crystal sample holders of either 1.0, 0.5 mm or 0.1 mm depth and 12 mm cavity diameter were used. The tube voltage and current were 40 kV and 40 mA, respectively. The X-ray diffractometer is equipped with a LynxEye detector. A variable divergence slight was used with a 3 ° window. The step size was 0.02 °2θ with a step time of 37 seconds. The samples were rotated at 0.5 rps during the measurement.

### Thermogravimetry coupled with Fourier Transform Infrared Spectroscopy (TG-FTIR)

TG-FTIR was performed on a Netzsch Thermo-Microbalance TG 209 which is coupled to a Bruker FT-IR Spectrometer Vector 22. The measurements were carried out with aluminum crucibles with a micro pinhole under a nitrogen atmosphere and at a heating rate of 10 °C/min over the range 25-250 °C.

### FT-Raman spectroscopy

Raman spectra were recorded using a Bruker RFS100 Raman spectrometer equipped with a germanium detector and a Nd:YAG laser with an excitation wavelength of 1064 nm. A few milligrams of material were pressed into aluminum sample holders. Spectra in the range of 50-3500 cm⁻¹ and with a resolution of 2 cm⁻¹ were measured with a laser power of 300 mW. 64 scans were accumulated.

### HPLC

HPLC was carried out on a TSP HPLC chromatograph (UV3000, AS3000, P4000, SCM1000 software version 4.1). The column type used was a Waters XTerra MS C18, 100 x 4.6 mm, 5 µm (CC01C). Mobile phase A was H₂O / ACN 95:5 + 0.1 % TFA and mobile phase B was H₂O / ACN 5:95 + 0.1 % TFA. The applied flow rate was 1.0 mL per minute, the injection volume was 10 microliter and the detection wavelength was 240 nm. The gradient was at 0 min 100% mobile phase A, at 20 min 100% mobile phase B, from 20 to 30 minutes pure mobile phase A.

### ¹H-NMR

The ¹H-NMR spectra were recorded using a Bruker DPX300 instrument. Generally, DMSO-d₆ was used as the solvent.

### Example 1

Preparation of the crystalline compound comprising Tiotropium bromide and propionic acid from the amorphous form.

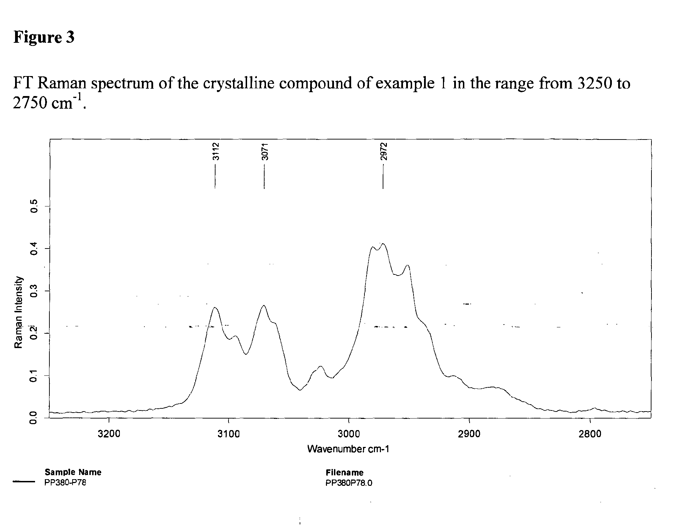

To about 300 mg of amorphous tiotropium bromide is added 5.0 ml propionic acid (Fluka # 49916) and the resulting suspension is stirred overnight at room temperature. The obtained crystalline solid is separated by filtration and dried at room temperature in air at a low relative humidity of about 20-to-30% for about 30 minutes. Investigation by powder X-ray diffraction shows that a XRPD pattern which is obtained and further investigation by FT Raman spectroscopy shows that this crystalline form also has a characteristic Raman spectrum. The XRPD pattern is shown in Figure 1 and the corresponding peak list is provided in Table 1. The Raman spectrum is depicted in Figure 2 and Figure 3 and the corresponding peak list is provided in Table 2. ¹H-NMR spectroscopy confirms the identity of tiotropium bromide and is consistent with the presence of about 0.5 equivalents of propionic acid in the obtained crystalline compound.

**Table 1: Powder X-ray diffraction peaks (M038)**

| **Pos. [°2θ.]** | **d-spacing [Å]** | **Qualitative Intentsity** |
|---|---|---|
| 6.7 | 13.1 | VW |
| 9.8 | 9.0 | w |
| 11.0 | 8.0 | m |
| 12.8 | 6.9 | vw |
| 13.3 | 6.6 | m |
| 13.5 | 6.6 | w |
| 14.6 | 6.1 | w |
| 15.1 | 5.87 | m |
| 15.3 | 5.77 | s |
| 16.1 | 5.51 | w |
| 16.2 | 5.47 | m |
| 17.8 | 4.99 | w |
| 18.0 | 4.93 | vs |
| 19.7 | 4.50 | s |
| 19.9 | 4.46 | m |
| 20.3 | 4.37 | w |
| 20.8 | 4.28 | s |
| 21.2 | 4.19 | s |
| 21.5 | 4.14 | vs |
| 22.3 | 3.98 | w |
| 22.5 | 3.95 | w |
| 22.7 | 3.91 | w |
| 23.0 | 3.87 | w |
| 23.5 | 3.78 | s |
| 23.6 | 3.76 | w |
| 24.0 | 3.71 | s |
| 24.4 | 3.65 | m |
| 24.7 | 3.60 | s |
| 25.1 | 3.55 | s |
| 25.9 | 3.44 | s |
| 26.0 | 3.42 | w |
| 26.6 | 3.35 | w |
| 26.9 | 3.31 | s |
| 27.2 | 3.28 | m |
| 27.6 | 3.23 | m |
| 27.8 | 3.20 | m |
| 29.0 | 3.08 | w |
| 29.5 | 3.03 | m |
| 29.8 | 3.00 | m |
| 30.0 | 2.98 | w |
| 30.2 | 2.95 | s |
| 30.5 | 2.93 | m |
| 30.7 | 2.91 | m |
| 31.1 | 2.87 | w |
| 31.3 | 2.85 | w |
| 31.6 | 2.83 | w |
| 31.9 | 2.81 | s |
| 32.5 | 2.75 | w |
| 32.7 | 2.74 | w |
| 33.0 | 2.71 | m |
| 33.3 | 2.69 | m |
| 33.4 | 2.68 | w |
| 33.6 | 2.67 | w |
| 33.8 | 2.65 | w |
| 34.2 | 2.62 | m |
| 34.4 | 2.60 | m |
| 34.6 | 2.59 | w |
| 35.3 | 2.54 | w |
| 35.7 | 2.51 | w |
| 36.0 | 2.49 | w |
| 36.7 | 2.45 | w |
| 36.8 | 2.44 | w |
| 37.3 | 2.41 | m |
| 38.2 | 2.35 | w |
| 39.0 | 2.31 | m |

**Table 2: Raman peaklist**

| **Wavenumbers [cm-1]** | **Relative intensity** |
|---|---|
| 3112 | 0.12 |
| 3071 | 0.21 |
| 3024 | 0.04 |
| 2972 | 0.41 |
| 2952 | 0.03 |
| 1747 | 0.06 |
| 1527 | 0.03 |
| 1480 | 0.04 |
| 1435 | 1.03 |
| 1347 | 0.15 |
| 1323 | 0.04 |
| 1244 | 0.11 |
| 1216 | 0.04 |
| 1201 | 0.03 |
| 1160 | 0.08 |
| 1070 | 0.28 |
| 1040 | 0.11 |
| 1016 | 0.05 |
| 1002 | 0.12 |
| 964 | 0.06 |
| 889 | 0.09 |
| 861 | 0.16 |
| 798 | 0.04 |
| 773 | 0.04 |
| 754 | 0.12 |
| 700 | 0.31 |
| 667 | 0.10 |
| 653 | 0.21 |
| 625 | 0.05 |
| 538 | 0.07 |
| 433 | 0.06 |
| 417 | 0.03 |
| 360 | 0.03 |
| 290 | 0.08 |
| 255 | 0.04 |
| 231 | 0.03 |
| 210 | 0.12 |
| 175 | 0.05 |
| 110 | 0.04 |

### Example 2

### Preparation of the crystalline compound comprising Tiotropium bromide and propionic acid

199 mg of anhydrous monoclinic crystalline tiotropium bromide according to EP 1 401 445 A1 is suspended in 2.0 ml propionic acid (Fluka # 49916), puriss p.a. and the resulting suspension is stirred at room temperature for three days. After three days the suspension is filtered and the obtained solid is dried under a weak nitrogen flow (about 10 ml/min) for one hour at room temperature. Then the sample is characterized by powder X-ray diffraction, TG-FTIR and ¹H-NMR. Powder X-ray diffraction shows that the obtained form is crystalline and has the same XRPD pattern as the crystalline compound obtained in example 1. TG-FTIR reveals a mass loss of about 8.2% which is attributable to loss of propionic acid. ¹H-NMR spectroscopy confirms the identity of tiotropium bromide and is consistent with the presence of about 0.5 equivalents of propionic acid.

### Example 3

### Chemical and physical stability of the compound of example 1 under accelerated conditions

About 20 mg of the crystalline compound according to example 1 are placed into an aluminium DSC sample pan. After open storage in an inert gas glove-box with nitrogen for about one hour at room temperature the sample pan is closed under nitrogen and stored at 40°C for two months. After two months, the sample pan is opened and the solid is investigated by ¹H-NMR spectroscopy, HPLC and powder X-ray diffraction. The powder X-ray diffraction pattern is identical with the XRPD pattern according to example 1. The HPLC does not indicate any chemical impurities and the obtained ¹H-NMR spectrum is consistent with pure tiotropium bromide containing about 0.5 equivalents of propionic acid.

The pharmaceutical compositions comprising pharmaceutically acceptable, nontoxic and a therapeutically effective amount of the new solvate of tiotropium bromide of the present invention and also preparation methods thereof are the characteristic features of the present invention.

The pharmaceutical compositions comprising the solvate of tiotropium bromide of the present invention are in dry powder or pressurized metered dose inhalation composition forms, preferably in dry powder inhalation composition form.

During preparation process of dry powder inhalation compositions, two methods are commonly implemented in order to transmit effective amount of medicament to the target area. One of them is based on controlled agglomeration of undiluted medicament; the other one based on adhesion of micronised medicament particles to the surface of an inert carrier having large particle size. The pharmaceutical compositions of the present invention are preferably prepared by implementing the both methods, preferably by implementing the second method. When the second method is implemented, the pharmaceutical composition comprises at least one pharmaceutically acceptable inert carrier and optionally at least one pharmaceutically acceptable excipient different from the carrier(s) along with the active substance.

The term "micronised medicament particles" refers to propionic acid solvate of tiotropium bromide. The solvate of tiotropium bromide of the present invention is characterized by having an average particle size in the range of 1-10µm, preferably in the range of 1-5µm. The pharmaceutical compositions of the present invention are characterized by comprising propionic acid solvate of tiotropium bromide of the present invention in the range of 0.001 50%, preferably in the range of 0.01-10%.

The term "inert carrier" refers to lactose, more preferably lactose monohydrate for dry powder inhalation compositions of the present invention. The pharmaceutical compositions of the present invention can comprise at least one inert carrier having large particle size and at least one inert carrier having small particle size and optionally at least one excipient together. The inert carrier having large particle size of the present invention is characterized by having an average particle size (d₅₀) in the range of 10-350 µm, alternatively in the range of 10-250 µm, preferably in the range of 10-150 µm, more preferably of 150 µm; the inert carrier having small particle size of the present invention, on the other hand, is characterized by having an average particle size (d₅₀) in the range of 1-10 µm, preferably of 10 µm. The inert carriers having large particle size and small particle size can be the same or different compounds.

At least one pharmaceutically acceptable excipient can be selected from carbohydrates such as lactose, glucose, fructose, galactose, sucrose, maltose, trehalose, maltodextrins, dextrans, cyclodextrins, starch and cellulose; polyalcohols such as sorbitol, mannitol and xylitol; amino acids such as glycine, arginine, lysine, aspartic acid and glutamic acid; peptides such as human serum albumin; gelatine; various salts and taste masking agents. Said at least one excipient is not limited to these substances.

In preparation process of pressurized metered-dose inhalation compositions, two formulation strategies are implemented depending on physicochemical characteristics of the active substance and propellant gas system. One of them is solution, the other one is suspension formulation. The pharmaceutical composition preferred comprises propellant gases, surface active agents and at least one basic excipient selected from the group of co-solvents and optionally at least one other pharmaceutically acceptable excipient along with the active substance.

The term "active substance" refers to propionic acid solvate of tiotropium bromide. The solvate of tiotropium bromide of the present invention is characterized by having an average particle size in the range of 1-10µm, preferably in the range of 1-5µm. The pharmaceutical compositions of the present invention are characterized by comprising the propionic acid solvate of tiotropium bromide in the range of 0.001-50%, preferably in the range of 0.01-10%.

Depending on formulation strategy, at least one pharmaceutically acceptable excipient can be selected from propellant gases (propellants) such as chlorofluorocarbons, hydrofluoroalkanes and hydrocarbons; surface active agents (surfactants) such as oleic acid, polysorbates, propylene glycol, polyethylene glycol, cetyl alcohol, stearyl alcohol, sorbitan fatty acid esters, sugar esters of fatty acids, glycerides of fatty acids, isopropyl myristate and lecithin; cosolvents such as ethanol, water and diethyl ether; antioxidants such as butylated hydroxyanisole (BHA), sodium ascorbate, butylated hydroxytoluene (BHT), sodium sulphide, gallates (such as propyl gallate), tocopherol, citric acid, malic acid, ascorbic acid, acetylcysteine, fumaric acid, lecithin, ascorbyl palmitate, ethylenediamine tetraacetate; and sweeteners.

The pharmaceutical compositions comprising the solvate of tiotropium bromide of the present invention can additionally comprise at least one active substance selected from the medicaments such as other anticholinergic agents, adrenergic agonists, antiallergic agents, anti-inflammatory agents, antihistaminics, steroids, leukotriene receptor antagonists, antimuscarinic agents, PDE inhibitors and EGFR inhibitors. The solvate of tiotropium bromide of the present invention can be used separately, sequentially or simultaneously with at least one active substance selected from the specified group.

Another characteristic feature of the present invention is that the pharmaceutical compositions comprising the solvate of tiotropium bromide of the present invention are used in treatment of respiratory complaints and particularly COPD (chronic obstructive pulmonary disease) and asthma.

### Preparation methods for the pharmaceutical compositions comprising propionic acid solvate of tiotropium bromide of the present invention

Preparation process of dry powder inhalation compositions of the present invention is characterized by comprising the following steps:
- sieving inert carriers having large particle size and small particle size separately,
- obtaining the first mixture as a result of adding the inert carrier sieved having small particle size into tiotropium bromide propionic acid solvate,
- sieving the first mixture obtained,
- obtaining the second mixture as a result of adding the inert carrier sieved having large particle size into the first mixture,
- sieving and mixing the second mixture obtained,
- making ready the final mixture to be filled into capsule/blister

Preparation process of the pressurized metered-dose inhalation compositions of the present invention is characterized by comprising the following steps:
- cooling production vessel to -25°C,
- pumping propellant gas into the vessel,
- adding tiotropium bromide propionic acid solvate into the vessel and mixing the mixture obtained,
- filling the final mixture into appropriate cups.

## Claims

1. A crystalline compound comprising a mixture of a compound of formula I (Tiotropium bromide) 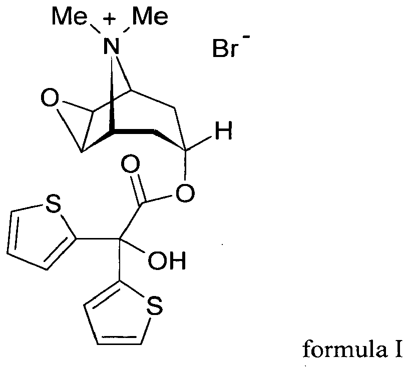 and propionic acid or a hydrate thereof, wherein the molar ratio of the compound of formula I and the propionic acid is in the range of from 1: 0,3 to 1:0,7.

2. The crystalline compound according to claim 1, **characterized in that** the molar ratio of the compound of formula I and the propionic acid is in the range of from 1: 0.45 to 1:0.55.

3. The crystalline compound according claim 1 or 2, **characterized in that** it has a FT-Raman spectrum comprising peaks at wavenumbers (expressed in ± 2 cm⁻¹) of 3112, 3071, 2792, 1747, 1435, 1347, 1244, 1160, 1070, 1040, 964, 889, 861, 754, 700, 667, 653, 538, 433, 290 and 210 cm⁻¹.

4. The crystalline compound according to at least one of the claims 1 to 3, **characterized in that** it has an XRPD pattern with at least one characteristic peak (expressed in 2θ ± 0,1° 2θ (CuKα radiation)) at 13.3°, 15.2°, 19.7°, 20.8°, 21.2°, 21.5°, 24.0°, 25.1°, 27.6°, 30.3°, 31.9° and 39.5°.

5. A pharmaceutical composition comprising the crystalline compound according to at least one of the claims 1 to 4, and optionally one or more pharmaceutically acceptable excipients.

6. The pharmaceutical composition according to claim 5, wherein the crystalline compound has an average particle size in the range of 1 to 10 µm.

7. The pharmaceutical composition according to claim 5, wherein said composition comprises crystalline compound in the range of 0.001-50 % in proportion to total weight of unit dose.

8. The pharmaceutical composition according to claim 5, wherein said composition is in dry powder form or pressurized metered dose form.

9. The pharmaceutical composition according to claim 8, wherein in the case that said composition is in dry powder form, it comprises at least one pharmaceutically acceptable inert carrier and optionally at least one pharmaceutically acceptable excipient different from the carrier used optionally along with the active substance.

10. The pharmaceutical composition according to claim 9, wherein the inert carrier is lactose.

11. The pharmaceutical composition according to claim 9, wherein the inert carrier comprises at least one inert carrier having large particle size and at least one inert carrier having small particle size, wherein the inert carrier having large particle size is **characterized by** having an average particle size (d₅₀) in the range of 10-350µm, and the inert carrier having small particle size is **characterized by** having an average particle size in the range of 1-10µm.

12. The pharmaceutical composition according to claim 5, wherein said composition can comprise at least one active substance selected from the medicaments such as other anticholinergic agents, adrenergic agonists, antiallergic agents, anti-inflammatory agents, antihistaminics, steroids, leukotriene receptor antagonists, antimuscarinic agents, PDE inhibitors and EGFR inhibitors.

13. A process for obtaining the crystalline compound according to at least one of the claims 1 to 4 comprising the steps of:
a) providing a compound of formula I (Tiotropium bromide) 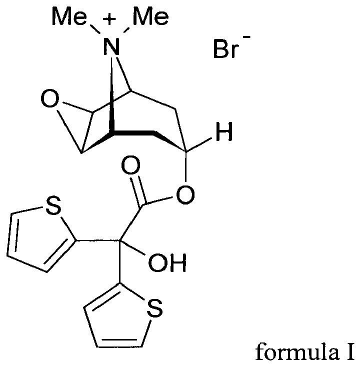
b) adding propionic acid to the composition of step a)
c) optionally concentrating the composition of step b)
d) crystallizing
e) optionally equilibrating the obtained suspension of step d)
f) isolating the obtained precipitate.

14. The process according to claim 6, **characterized in that** seed crystals are added in step d) and/or in step e).

## Patentansprüche

1. Eine kristalline Verbindung, die ein Gemisch aus einer Verbindung der Formel I (Tiotropium Bromid) und Propionsäure oder ein Hydrat davon umfasst, 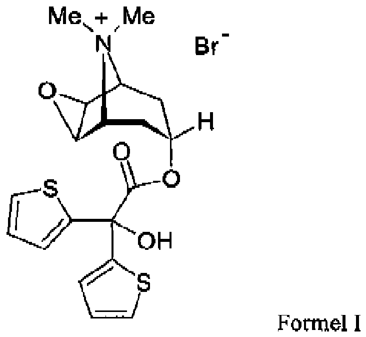 wobei das molare Verhältnis der Verbindung der Formel I und der Propionsäure im Bereich von 1: 0,3 bis 1: 0,7 liegt.

2. Die kristalline Verbindung nach Anspruch 1, **dadurch gekennzeichnet, dass** das molare Verhältnis der Verbindung der Formel I und der Propionsäure im Bereich von 1: 0,45 bis 1: 0,55 liegt.

3. Die kristalline Verbindung nach Anspruch 1 oder 2, **dadurch gekennzeichnet, dass** sie ein FT-Raman-Spektrum enthaltend Spitzen bei Wellenzahlen (in ± 2 cm⁻¹ ausgedrückt wird) von 3112 , 3071, 2792, 1747 1435, 1347, 1244, 1160, 1070, 1040, 964, 889, 861, 754, 700, 667, 653, 538,433, 290 und 210 cm⁻¹ hat.

4. Die kristalline Verbindung nach mindestens einem der Ansprüche 1 bis 3, **dadurch gekennzeichnet, dass** sie einen XRPD-Muster mit mindestens einer charakteristischen Spitze (in 2θ ± 0,1° 2θ (CuKα Radiation) ausgedrückt wird) bei 13.3°, 15.2°, 19.7°, 20.8°, 21.2°, 21.5°, 24.0°, 25.1°, 27.6°, 30.3°, 31.9° und 39.5° hat.

5. Eine pharmazeutische Zusammensetzung, die die kristalline Verbindung nach mindestens einem der Ansprüche 1 bis 4 und gegebenenfalls einen oder mehreren pharmazeutisch annehmbaren Hilfsstoffen umfasst.

6. Die pharmazeutische Zusammensetzung nach Anspruch 5, wobei die kristalline Verbindung eine durchschnittliche Teilchengröße im Bereich von 1 bis 10 µm hat.

7. Die pharmazeutische Zusammensetzung nach Anspruch 5, wobei die genannte Zusammensetzung kristalline Verbindung im Bereich von 0,001-50% im Verhältnis zum Gesamtgewicht der Einheitsdosis enthält.

8. Die pharmazeutische Zusammensetzung nach Anspruch 5, wobei die genannte Zusammensetzung in trockener Pulverform oder unter Druck stehender dosierter Form liegt.

9. Die pharmazeutische Zusammensetzung nach Anspruch 8, wobei in dem Fall, dass die genannte Zusammensetzung in Trockenpulverform liegt, enthält sie mindestens einen pharmazeutisch annehmbaren inerten Träger und gegebenenfalls mindestens einen pharmazeutisch annehmbaren Hilfsstoff, der sich vom gegebenenfalls zusammen mit dem Wirkstoff verwendeten Träger unterscheidet.

10. Die pharmazeutische Zusammensetzung nach Anspruch 9, wobei der inerte Träger Lactose ist.

11. Die pharmazeutische Zusammensetzung nach Anspruch 9, wobei der inerte Träger mindestens einen inerten Träger mit großer Teilchengröße und mindestens einen inerten Träger mit kleiner Teilchengröße enthält, bei dem der inerte Träger mit der großen Teilchengröße durch eine durchschnittliche Teilchengröße (d50) im Bereich von 10-350µm, und der inerte Träger mit kleiner Teilchengröße durch eine durchschnittliche Teilchengröße im Bereich von 1-10µm gekennzeichnet wird.

12. Die pharmazeutische Zusammensetzung nach Anspruch 5, wobei die genannte Zusammensetzung mindestens einen Wirkstoff enthält, der aus den Medikamenten wie andere anticholinerge Mittel, adrenergische Agonisten, antiallergische Mittel, entzündungshemmende Mittel, Antihistaminika, Steroide, Leukotrienrezeptor-Antagonisten, antimuskarinische Wirkstoffe , PDE-Inhibitoren und EGFR-Inhibitoren ausgewählt wird.

13. Verfahren zur Gewinnung der kristallinen Verbindung nach mindestens einem der Ansprüche von 1 bis 4, der die folgenden Schritte umfasst:
a) Bereitstellen einer Verbindung der Formel I (Tiotropiumbromid) 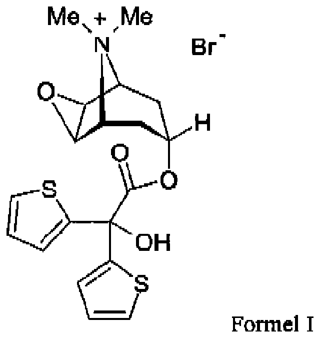
b) Zugabe von Propionsäure zu der Zusammensetzung von Schritt a)
c) gegebenenfalls Konzentrieren der Zusammensetzung von Schritt b)
d) Kristallisieren
e) gegebenenfalls Abgleichen der erhaltenen Suspension von Schritt d)
f) Isolieren des erhaltenen Niederschlags.

14. Das Verfahren nach Anspruch 6, **dadurch gekennzeichnet, dass** Impfkristalle im Schritt d) und / oder im Schritt e) hinzugefügt werden.

## Revendications

1. Un composé cristallin comprenant un mélange d'un composé de formule 1 (le bromure de tiotropium) 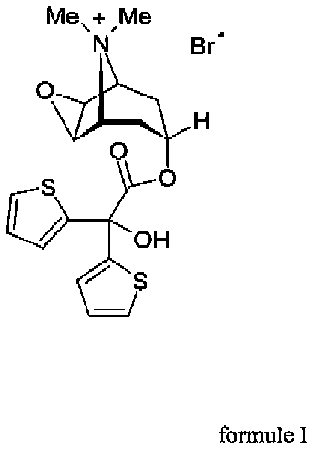 et l'acide propionique ou un de ses hydrates, dans lequel le rapport molaire du composé de formule 1 et l'acide propionique est dans la plage de 1:0,3 à 1:0,7.

2. Le composé cristallin selon la revendication 1, **caractérisé en ce que** le rapport molaire du composé de formule I et l'acide propionique est dans la plage de 1:0,45 à 1:0,55.

3. Le composé cristallin selon la revendication 1 ou 2, **caractérisé en ce qu'**il a un spectre FT-Raman, comprenant des pics à des nombres d'onde (exprimé en cm ± 2 cm-1) de 3112, 3071, 2792, 1747, 1435, 1347, 1244, 1160, 1070, 1040, 964, 889, 861, 754, 700, 667, 653, 538, 433, 290 et 210 cm-1.

4. Le composé cristallin selon au moins l'une des revendications 1 à 3, **caractérisé en ce qu'**il a un spectre XRPD avec au moins un pic caractéristique (exprimé en 2θ ± 0,1° 2θ (CuKα rayonnement)) à 13.3°, 15.2°, 19.7°, 20.8°, 21.2°, 21.5°, 24.0°, 25.1°, 27.6°, 30.3°, 31.9° et 39.5°.

5. Une composition pharmaceutique comprenant le composé cristallin selon au moins l'une des revendications 1 à 4, et éventuellement un ou plusieurs excipients pharmaceutiquement acceptables.

6. La composition pharmaceutique selon la revendication 5, dans laquelle le composé cristallin a une taille moyenne des particules dans la plage de 1 à 10 µm.

7. La composition pharmaceutique selon la revendication 5, dans laquelle ladite composition comprend un composé cristallin dans la plage de 0.001 à 50% en proportion de poids total de la dose unitaire.

8. La composition pharmaceutique selon la revendication 5, dans laquelle ladite composition est sous la forme de poudre sèche ou sous la forme de la dose mesurée sous pression.

9. La composition pharmaceutique selon la revendication 8, dans laquelle, dans le cas où ladite composition est sous la forme de poudre sèche, elle comprend au moins un porteur inerte pharmaceutiquement acceptable et éventuellement au moins un excipient pharmaceutiquement acceptable différente de la porteur utilisée éventuellement avec le principe actif.

10. La composition pharmaceutique selon la revendication 9, dans laquelle le porteur inerte est le lactose.

11. La composition pharmaceutique selon la revendication 9, dans laquelle le porteur comprend au moins un porteur inerte ayant une grande taille des particules et au moins un porteur inerte ayant une petite taille des particules, dans laquelle le porteur inerte ayant une grande taille des particules est **caractérisée par** ayant une taille de particule moyenne (d₅₀) dans la plage de 10-350µm, et le porteur inerte ayant une petite taille des particules est **caractérisée par** ayant une taille de particule moyenne dans la plage de 1-10µm.

12. La composition pharmaceutique selon la revendication 5, dans laquelle ladite composition peut comprendre au moins un principe actif choisi parmi les médicaments tels que d'autres agents anti-cholinergiques, les agonistes adrénergiques, les agents antiallergiques, les agents anti-inflammatoires, les antihistaminiques, les stéroïdes, les antagonistes des récepteurs des leucotriènes, les agents antimuscariniques, les inhibiteurs de PDE, et les inhibiteurs de l'EGFR.

13. Un procédé pour l'obtention du composé cristallin selon au moins l'une des revendications 1 à 4, comprenant les étapes suivantes:
a) fournir un composé de formule I (le bromure de tiotropium) 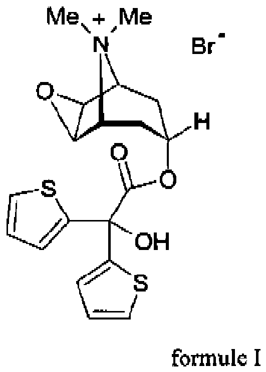
b) ajouter de l'acide propionique à la composition de l'étape a)
c) concentrer éventuellement la composition de l'étape b)
d) cristalliser
e) équilibrer éventuellement la suspension obtenue de l'étape d)
f) isoler le précipité obtenu.

14. Le procédé selon la revendication 6, **caractérisé en ce que** les cristaux d'ensemencement sont ajoutés durant l'étape d) et/ou durant l'étape e).
